# EUROPEAN PATENT APPLICATION

(11) **EP 2 266 463 A1**
(43) Date of publication of application: **29.12.2010**
(21) Application number: 09728576.1
(22) Date of filing: 27.03.2009
(51) Int. Cl.: A61B 5/157

(54) **METHOD OF ANALYZING ORGANISM COMPONENTS, ORGANISM COMPONENT ANALYZER, AND EXTRACTION CARTRIDGE**

(30) Priority: 31.03.2008 JP 2008091599
(71) Applicant: Sysmex Corporation, Kobe-shi, Hyogo 651-0073 (JP); Kabushiki Kaisha Toshiba, Minato-ku Tokyo 105-8001 (JP)
(72) Inventor: KOJIMA, Junko, Kobe-shi Hyogo 651-0073 (JP); MAEKAWA, Yasunori, Kobe-shi Hyogo 651-0073 (JP); TAKASE, Tomohiro, Tokyo 105-8001 (JP); UEMATSU, Ikuo, Tokyo 105-8001 (JP); HIRAKAWA, Masaaki, Tokyo 105-8001 (JP); NAWATA, Isao, Tokyo 105-8001 (JP); TONO, Ichiro, Tokyo 105-8001 (JP); KASAI, Shingo, Tokyo 105-8001 (JP)
(74) Representative: HOFFMANN EITLE
(86) International application number: PCT/JP2009/056243
(87) International publication number: WO 2009/123036

(57) **Abstract**

This method of analyzing a biological component includes steps of arranging an extraction medium holding part configured to be capable of holding a prescribed quantity of extraction medium for holding an extract extracted from a subject on the skin of the subject, transferring the extraction medium stored in a liquid storing part storing substantially the same quantity of the extraction medium as the prescribed quantity to the extraction medium holding part, transferring at least part of the extraction medium holding the extracted extract to a reaction part, and analyzing a result of detecting the extract.

## Description

### Technical Field

The present invention relates to a method of analyzing a biological component, a biological component analyzer and an extraction cartridge.

### Background Art

A biological component analyzer reacting extracted glucose to analyze the same by employing an enzyme such as glucose oxidase as a catalyst after extracting an analyte such as glucose from a subject is known in general.

In the conventional biological component analyzer, however, the enzyme such as glucose oxidase is eluted into extraction medium for holding the glucose extracted from the subject before the same functions as a catalyst for the glucose, and consequently there is a possibility that analytical accuracy of the glucose is reduced.

In this regard, a biological component analyzer capable of suppressing the analytical accuracy of the glucose from reduction is known in general. Such a biological component analyzer is disclosed in Japanese Patent Laying-Open No. 2005-246054, for example.

Japanese Patent Laying-Open No. 2005-246054 discloses a biological component analyzer comprising a reaction part reacting glucose by employing glucose oxidase as a catalyst, an extracting part configured to be capable of holding an extraction medium for holding glucose extracted from a subject and a syringe pump storing the extraction medium and supplying (transferring) the same to each part. In this biological component analyzer, the syringe pump supplies the stored extraction medium to the extracting part and further supplies the extraction medium to the extracting part after the glucose is extracted to the extraction medium in the extracting part thereby transferring (ejecting) the extraction medium holding the glucose from the extracting part to the reaction part. Thus, the reaction part can be held in a dry state until the extraction medium holding the glucose is transferred to the reaction part, and hence the glucose oxidase in the reaction part can be suppressed from elution into the extraction medium. Consequently, analytical accuracy of the glucose can be suppressed from reduction.

In the biological component analyzer described in Japanese Patent Laying-Open No. 2005-246054, however, the reaction part is held in a dry state until the extraction medium holding the glucose is transferred to the reaction part so that the analytical accuracy of the glucose can be suppressed from reduction, while a liquid supplying mechanism further supplies the extraction medium from the syringe pump to the extracting part after the glucose is extracted to the extraction medium in the extracting part thereby transferring (ejecting) the extraction medium holding the glucose from the extracting part to the reaction part, and hence the extraction medium holding the glucose in the extracting part comes into contact with the extraction medium from the syringe pump for ejecting the extraction medium holding the glucose in the extracting part to the reaction part and there is a possibility that the glucose is mixed with the extraction medium from the syringe pump. When the glucose is mixed with the extraction medium outside the extracting part, the concentration of the glucose in the extraction medium becomes non-uniform and the analytical accuracy may be reduced.

### Disclosure of the Invention

The present invention has been proposed in order to solve the aforementioned problems, and an object of the present invention is to provide a method of analyzing a biological component, a biological component analyzer and an extraction cartridge allowing for a more accurate analysis of an extract extracted from a subject.

In order to attain the aforementioned object, a method of analyzing a biological component according to a first aspect of the present invention comprises steps of arranging an extraction medium holding part configured to be capable of holding a prescribed quantity of extraction medium for holding an extract extracted from a subject on the skin of the subject, transferring the extraction medium stored in a liquid storing part storing substantially the same quantity of the extraction medium as the prescribed quantity to the extraction medium holding part, extracting the extract to the extraction medium transferred to the extraction medium holding part, transferring at least part of the extraction medium holding the extracted extract to a reaction part allowing the extract to cause a prescribed reaction, detecting the extract from the prescribed reaction caused in the reaction part, and analyzing a result of detecting the extract.

In the aforementioned method of analyzing a biological component according to the first aspect, the step of transferring the extraction medium to the extraction medium holding part preferably includes a step of transferring the extraction medium stored in the liquid storing part to the extraction medium holding part with a liquid transfer part.

In the aforementioned method of analyzing a biological component having the step of transferring the extraction medium stored in the liquid storing part with the liquid transfer part, the step of transferring the extraction medium with the liquid transfer part preferably includes a step of transferring the extraction medium stored in the liquid storing part to the extraction medium holding part by pressure of air by allowing air to flow in an entrance passage provided between the liquid transfer part and the liquid storing part and filled with air with the liquid transfer part.

In this case, the step of transferring the extraction medium to the extraction medium holding part with the liquid transfer part preferably includes a step of transferring substantially all of the extraction medium stored in the liquid storing part to the extraction medium holding part through an exit passage provided between the liquid storing part and the extraction medium holding part.

In the aforementioned method of analyzing a biological component according to the first aspect, the step of transferring at least the part of the extraction medium to the reaction part preferably includes a step of transferring at least the part of the extraction medium holding the extracted extract to the reaction part with the liquid transfer part.

The aforementioned method of analyzing a biological component according to the first aspect preferably further comprises a step of allowing a mesh sheet arranged in the reaction part to absorb the extraction medium transferred to the reaction part after the step of transferring at least the part of the extraction medium to the reaction part.

In the aforementioned method of analyzing a biological component according to the first aspect, the step of detecting the extract preferably includes a step of detecting glucose, and the step of analyzing the result of detecting the extract preferably includes a step of acquiring a blood glucose level.

A biological component analyzer according to a second aspect of the present invention comprises an extraction medium holding part capable of holding a prescribed quantity of extraction medium for holding an extract extracted from a subject, a reaction part allowing the extract to cause a prescribed reaction, a detecting part detecting the extract from the prescribed reaction caused in the reaction part, a liquid storing part storing substantially the same quantity of the extraction medium as the prescribed quantity, a liquid transfer part transferring the extraction medium stored in the liquid storing part to the extraction medium holding part and transferring at least part of the extraction medium transferred to the extraction medium holding part, holding the extract extracted from the subject from the extraction medium holding part to the reaction part, and an analysis part analyzing a result of detecting the extract, and is configured to allow for extraction of the extract to the extraction medium holding part by arranging the extraction medium holding part to be in contact with the skin of the subject.

In the aforementioned biological component analyzer according to the second aspect, the prescribed quantity of the extraction medium capable of being held by the extraction medium holding part is preferably larger than the quantity of the extraction medium capable of being held by the reaction part.

The aforementioned biological component analyzer according to the second aspect preferably further comprises an entrance passage and an exit passage each filled with gas and connected to the liquid storing part.

In this case, the gas is preferably air, and the biological component analyzer preferably further comprises an air inflow part for allowing air to flow in the entrance passage and an ejection part configured to be capable of blocking the air inflow part, ejecting air supplied from the liquid transfer part to the air inflow part.

The aforementioned biological component analyzer according to the second aspect preferably further comprises a cartridge including the extraction medium holding part, the reaction part and the liquid storing part and an analyzer body including the detecting part, the liquid transfer part and the analysis part, configured to allow the cartridge to be attached and detached.

The aforementioned biological component analyzer according to the second aspect preferably further comprises an extraction promoting part promoting extraction of the extract.

The aforementioned biological component analyzer according to the second aspect preferably further comprises an intrusion prevention part preventing the skin of the subject from intrusion into the extraction medium holding part.

The aforementioned biological component analyzer according to the second aspect preferably further comprises a mesh sheet arranged in the reaction part, capable of absorbing at least part of the extraction medium transferred to the reaction part.

In the aforementioned biological component analyzer according to the second aspect, the extract is preferably glucose, and the analysis part is preferably configured to estimate a blood glucose level by analyzing a result of detecting the glucose.

A biological component analyzer according to a third aspect of the present invention comprises an extraction medium holding part holding a prescribed quantity of extraction medium for holding an extract extracted from a subject, an entrance passage and an exit passage filled with gas, connected to the extraction medium holding part, a reaction part allowing the extract to cause a prescribed reaction, a detecting part detecting the extract from the prescribed reaction caused in the reaction part, a liquid transfer part transferring at least part of the extraction medium holding the extract extracted from the subject from the extraction medium holding part to the reaction part through the exit passage, and an analysis part analyzing a result of detecting the extract, and is configured to allow for extraction of the extract to the extraction medium holding part by arranging the extraction medium holding part to be in contact with the skin of the subject.

An extraction cartridge according to a fourth aspect of the present invention comprises an extraction medium holding part configured to be capable of holding a prescribed quantity of extraction medium for holding an extract extracted from a subject, a reaction part allowing the extract to cause a prescribed reaction, and a liquid storing part storing substantially the same quantity of the extraction medium as the prescribed quantity, and is detachable on an analyzer body detecting the extract from the prescribed reaction caused in the reaction part to analyze the same.

The aforementioned extraction cartridge according to the fourth aspect preferably further comprises a mesh sheet arranged in the reaction part, capable of absorbing at least part of the extraction medium transferred to the reaction part.

An extraction cartridge according to a fifth aspect of the present invention comprises an extraction medium holding part holding a prescribed quantity of extraction medium for holding an extract extracted from a subject, an entrance passage and an exit passage filled with gas, connected to the extraction medium holding part, and a reaction part allowing the extract to cause a prescribed reaction, and is detachable on an analyzer body detecting the extract from the prescribed reaction caused in the reaction part to analyze the same.

### Brief Description of the Drawings

[Fig. 1] A perspective view showing a blood glucose level estimation device according to an embodiment of the present invention.
[Fig. 2] A perspective view showing a state where a device body of the blood glucose level estimation device according to the embodiment shown in Fig. 1 is opened.
[Fig. 3] A perspective view showing a state where an analytic unit of the blood glucose level estimation device according to the embodiment shown in Fig. 1 is mounted on the arm of a subject.
[Fig. 4] A schematic diagram showing the structure of the blood glucose level estimation device according to the embodiment shown in Fig. 1.
[Fig. 5] A plane view of an extraction cartridge of the blood glucose level estimation device according to the embodiment shown in Fig. 1 as viewed from above.
[Fig. 6] A plane view of the extraction cartridge of the blood glucose level estimation device according to the embodiment shown in Fig. 1 as viewed from below.
[Fig. 7] A sectional view taken along the line 200-200 shown in Fig. 5.
[Fig. 8] A flow chart for illustrating a procedure when the subject employs the blood glucose level estimation device.
[Fig. 9] A diagram for illustrating a use procedure of the blood glucose level estimation device according to the embodiment shown in Fig. 1.
[Fig. 10] A sectional view showing the skin on which micropores are formed by a micropore forming device shown in Fig. 9.
[Fig. 11] A schematic diagram showing the unused extraction cartridge employed in the blood glucose level estimation device according to the embodiment shown in Fig. 1.
[Fig. 12] A flow chart for illustrating measurement processing by a control part of the blood glucose level estimation device according to the embodiment shown in Fig. 1.
[Fig. 13] A plane view showing the extraction cartridge in a state where a liquid storing part stores liquid in the blood glucose level estimation device according to the embodiment shown in Fig. 1.
[Fig. 14] A sectional view showing the extraction cartridge in a state where the liquid storing part stores the liquid in the blood glucose level estimation device according to the embodiment shown in Fig. 1.
[Fig. 15] A plane view showing the extraction cartridge in a state where an extraction medium holding part holds the liquid in the blood glucose level estimation device according to the embodiment shown in Fig. 1.
[Fig. 16] A sectional view showing the extraction cartridge in a state where the extraction medium holding part holds the liquid in the blood glucose level estimation device according to the embodiment shown in Fig. 1.
[Fig. 17] A plane view showing the extraction cartridge in a state where a reaction part holds the liquid in the blood glucose level estimation device according to the embodiment shown in Fig. 1.
[Fig. 18] A sectional view showing the extraction cartridge in a state where the reaction part holds the liquid in the blood glucose level estimation device according to the embodiment shown in Fig. 1.

### Best Modes for Carrying Out the Invention

An embodiment of the present invention will be hereinafter described with reference to the drawings.

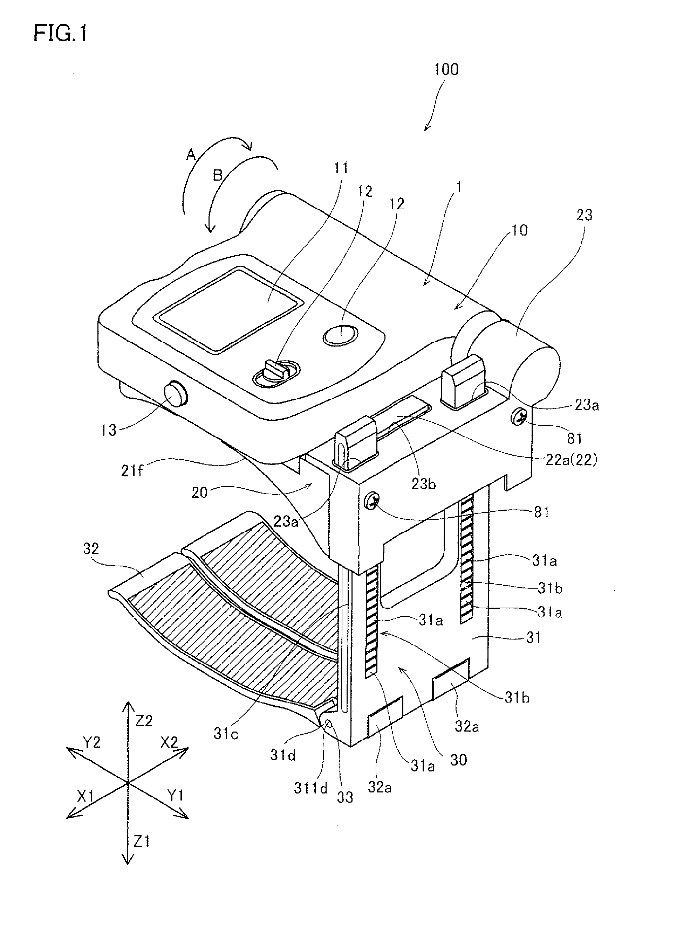

The structure of a blood glucose level estimation device 100 according to the embodiment will be now described with reference to Figs. 1 to 7.

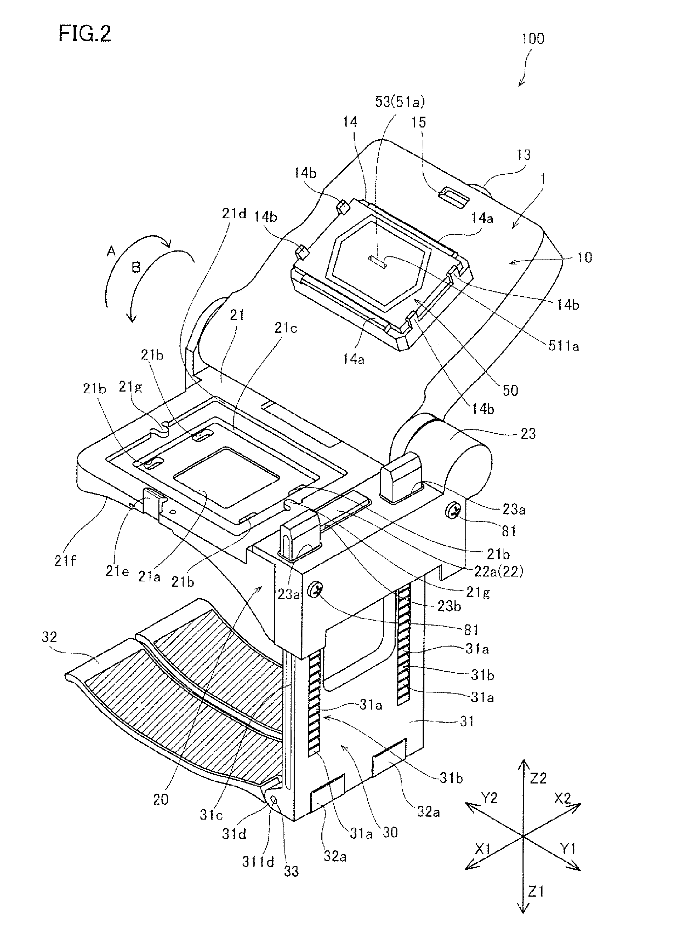

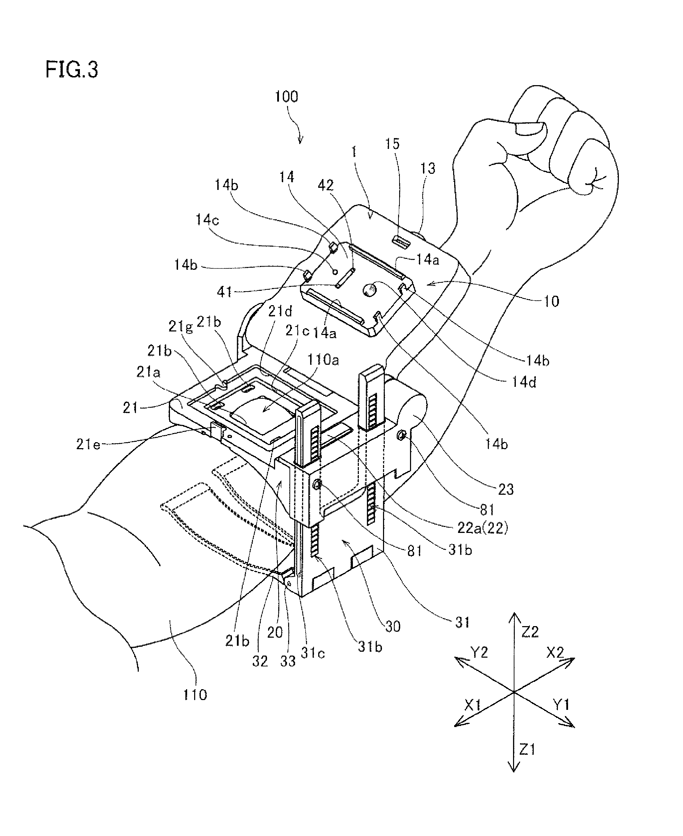

The blood glucose level estimation device 100 according to the embodiment is a device for extracting glucose, which is one of biochemical components, from a living body and estimating a blood glucose level by analyzing the extracted glucose. This blood glucose level estimation device 100 comprises an analytic unit 1 and an extraction cartridge 50 (see Fig. 2), as shown in Figs. 1 and 2. The analytic unit 1 includes an device body 10 detachably holding the extraction cartridge 50, a holding member 20 holding the device body 10 rotatably along arrow A and arrow B and a mounting member 30 for mounting the holding member 20 on the arm 110 (see Fig. 3) of a subject.

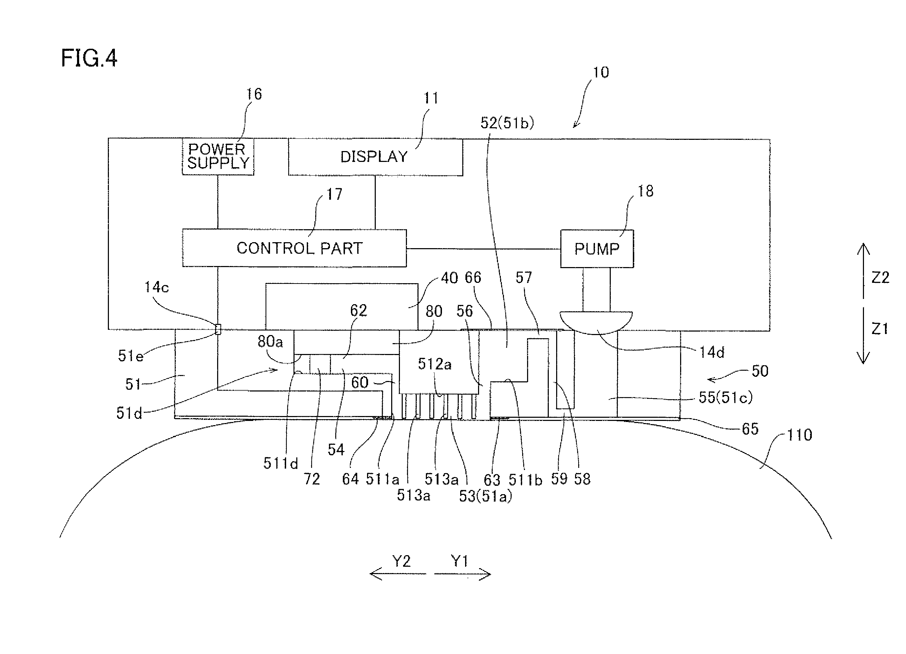

The device body 10 of the analytic unit 1 includes a display 11 (see Fig. 1) and operation buttons 12 (see Fig. 1) arranged on a front surface side, a release button 13 arranged on an end side in an arrow X1 direction in a state of use (state of closing the device body *10), an extraction cartridge attachment part 14 (see Fig. 2 and 3) and an engaging hole 15 (see Fig. 2 and 3) arranged on a back surface side, a power supply 16 (see Fig. 4) functioning as a direct-current system constant-voltage power supply and also an alternate-current system highfrequency power supply, provided internally a control part 17 estimating the amount of glucose and a blood glucose level based on a detection result of glucose by a detecting part 40 described later and a pump 18 supplying air, as shown in Figs. 1 to 3.

The display 11 of the device body 10 is provided to display the amount of glucose calculated and/or the blood glucose level estimated by the control part 17. The operation buttons 12 of the device body 10 are provided for the subject to operate the blood glucose level estimation device 100 such as to start measurement. The release button 13 of the device body 10 is provided to release engagement of an engaging part 21e of the holding member 20 described later and the engaging hole 15 (see Figs. 2 and 3) of the device body 10.

The extraction cartridge attachment part 14 of the device body 10 includes a pair of rib-shaped projecting parts 14a functioning as guides when attaching the extraction cartridge 50, four engaging parts 14b for attaching the extraction cartridge 50 (see Fig. 2), a terminal 14c (see Fig. 3) connected to the power supply 16 (see Fig. 4) of the device body 10, a light source part 41 and a photoreceptor 42 configuring the detecting part 40 (see Fig. 4) and an ejection part 14d ejecting air supplied from the pump 18 to the outside of the extraction cartridge attachment part 14, as shown in Figs. 2 and 3. The four engaging parts 14b of the extraction cartridge attachment part 14 are elastically deformably provided thereby allowing the extraction cartridge 50 (see Fig. 2) to be attached and detached. The ejection part 14d has a hemisphere shape and is urged by a spring member (not shown) to protrude to the outside of the extraction cartridge attachment part 14.

The engaging hole 15 of the device body 10 is provided to engage with the engaging part 21e of the holding member 20 when the device body 10 is in a closed state (see Fig. 1) with respect to the holding member 20.

The holding member 20 includes a holding member body 21 rotatably holding the device body 10, an engaging member 22 configuring a ratchet mechanism and a cover member 23 covering a first end side (end side in an arrow Y1 direction) of the holding member body 21, as shown in Figs. 2 and 3.

The holding member body 21 of the holding member 20 includes an opening 21a provided in the vicinity of a central part thereof in plan view, four spaces 21b, step parts 21c and 21d and the engaging part 21e provided on the end side in the arrow X1 direction. The holding member body 21 is formed of ABS resin or the like. A lower surface part 21f of the holding member body 21 is formed in an arc shape when viewed from a direction X2. Thus, contact with the arm 110 of the subject can be improved when the holding member 20 is mounted on the arm 110 of the subject.

The opening 21a of the holding member body 21 is formed in a square shape in plan view. The holding member 20 is mounted on the arm 110 of the subject by the mounting member 30 as shown in Fig. 3, whereby the opening 21a has a function of defining a measuring area 110a of the subject's arm 110. At this time, the measuring area 100a of the subjects arm 110 protrudes in an arrow Z2 direction through the opening 21a.

The four spaces 21b of the holding member body 21 are provided to store the respective four engaging parts 14b (see Figs. 2 and 3) of the device body 10 when the device body 10 is in a closed state (see Fig. 1) with respect to the holding member 20. The step part 21c of the holding member body 21 is provided to store the extraction cartridge attachment part 14 (see Figs. 2 and 3) of the device body 10 when the device body 10 is in a closed state (see Fig. 1) with respect to the holding member 20. The step part 21d of the holding member body 21 is formed with a pair of engaging projections 21g, as shown in Fig. 2. The step part 21d and the engaging projections 21g have a function of positioning a micropore forming device 120 described later when using the same.

The engaging part 21e (see Figs. 2 and 3) of the holding member body 21 engages with the engaging hole 15 (see Figs. 2 and 3) of the device body 10 when the device body 10 is in a closed state (see Fig. 1) with respect to the holding member 20 thereby having a function of limiting rotation of the device body 10 in a direction A (opening direction) with respect to the holding member body 21. At this time, the extraction cartridge 50 is in contact with a surface of the subject's arm 110 exposed from the opening 21a of the holding member body 21 as shown in Fig. 4, when the extraction cartridge 50 (see Fig. 2) is attached to the device body 10.

A sheet (not shown) made of a conductive material, electrically connected to the power supply 16 is attached to a lower surface part 21f of the holding member body 21. Thus, the holding member body 21 is configured to function as an electrode.

The engaging member 22 is formed of polyacetal resin and includes a pressing part 22a. The engaging member 22 configures the ratchet mechanism regulating a moving direction of the mounting member 30 with respect to the holding member 20 unidirectionally (in the arrow Z2 direction). The engaging member 22 has a release function of releasing regulation of the ratchet mechanism and is so configured that the regulation of the ratchet mechanism is released with respect to the mounting member 30 when pressing the pressing part 22a. When releasing the pressing by the pressing part 22a, on the other hand, the engaging member 22 is so configured that the mounting member 30 is regulated by the ratchet mechanism.

The cover member 23 of the holding member 20 includes a pair of passing holes 23a through which the mounting member 30 passes and a hole 23b in which the pressing part 22a of the engaging member 22 is arranged. The cover member 23 is formed of ABS resin or the like. The cover member 23 is mounted on the holding member body 21 with screws 81.

The mounting member 30 includes a coupling part 31 and two tightening parts 32 extending in an arrow Y2 direction from a lower end (end on an arrow Z1 direction side) of the coupling part 31, as shown in Figs. 1 and 2. The mounting member 30 is formed of ABS resin or the like (rigid body). A sheet (not shown) made of a conductive material, electrically connected to the power supply 16 is attached to an inner surface of the mounting member 30, similarly to the sheet attached to the lower surface part 21f of the holding member body 21. Thus, the mounting member 30 is configured to function as an electrode.

The coupling part 31 of the mounting member 30 includes a pair of engaging regions 31b having a plurality of engaging pawls 31a, a locking engaging groove 31c and a projection 31d protruding in the arrow Y2 direction. The coupling part 31 is so configured that a distance between the tightening parts 32 and the holding member 20 can be changed. The plurality of engaging pawls 31a are configured to allow the mounting member 30 to move in the arrow Z2 direction (upward) with respect to the holding member 20 and regulate the mounting member 30 to move in the arrow Z1 direction (downward) with respect to the holding member 20. The locking engaging groove 31c is provided to prevent the mounting member 30 from falling off the holding member 20 when slidingly moving the coupling part 31 in the arrow Z1 direction. The projection 31d protruding in the arrow Y2 direction is provided with a through-hole 311d extending in the arrow X1 direction and the arrow X2 direction.

The tightening parts 32 of the mounting member 30 are formed in the form of a curved plate when viewed from the arrow X2 direction and are elastically deformably formed. The tightening parts 32 are so configured that the subject's arm 110 (see Fig. 3) can be tightened between the tightening parts 32 and the holding member 20 from above and below by changing the distance between the tightening parts 32 and the holding member 20 by the coupling part 31. The tightening parts 32 are provided with two projecting parts 32a on an end on the arrow Y1 direction side. Each of the two projecting parts 32a is formed with a through-hole (not shown) extending in the arrow X1 direction and the arrow X2 direction. This through-hole is formed to be coaxial with the through-hole 311d of the coupling part 31, and a stainless-steel shaft member 33 is inserted into the through-hole 311d of the coupling part 31 and the through-hole of each of the projecting parts 32a. Thus, the tightening parts 32 can be rotated around the shaft member 33 with respect to the coupling part 31 and can be folded on a side of the coupling part 31.

According to the embodiment, the extraction cartridge 50 includes a cartridge body 51 made of acrylic resin or the like, a liquid storing part 52 storing pure water functioning as an extraction medium for holding glucose extracted from the subject's arm 110, an extraction medium holding part 53 to which glucose is extracted from the subject's arm 110, a reaction part 54 allowing glucose to cause a prescribed reaction and an air inflow part 55 in which air ejected from the ejection part 14d flows, as shown in Fig. 4.

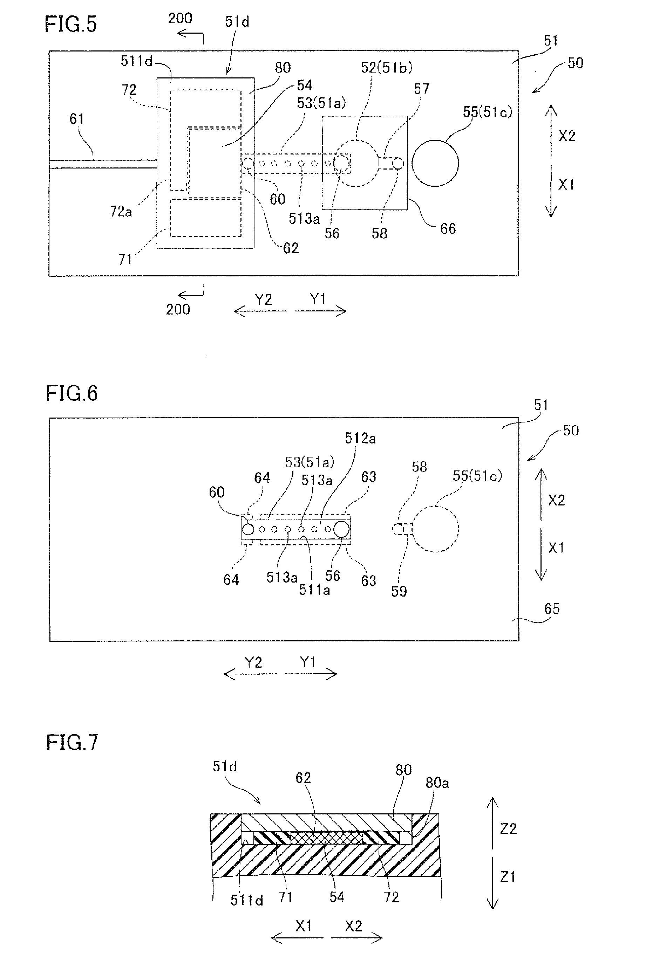

As shown in Figs. 4 and 6, a first recess part 51a having a rectangular opening 511a is formed on a substantially central part of a lower surface of the cartridge body 51. The extraction medium holding part 53 is configured by this first recess part 51a. The rectangular opening 511a is formed to extend in the arrow Y1 direction and the arrow Y2 direction. A plurality of columnar intrusion prevention parts 513a extending outward (in the arrow Z1 direction) are formed on a first bottom surface 512a in the first recess part 51a. The intrusion prevention parts 513a are so formed that a height thereof is substantially the same as a depth of the first recess part 51a. Therefore, forward end surfaces of the intrusion prevention parts 513a are located on substantially the same plane as the lower surface of the cartridge body 51. The plurality of intrusion prevention parts 513a are arranged in lines at prescribed intervals in the arrow Y1 direction and the arrow Y2 direction throughout the opening 511a. Thus, the surface of the subject's arm 110 can be prevented from intrusion into the extraction medium holding part 53. The extraction medium holding part 53 has a volume capable of storing a prescribed quantity (about 5 µl to about 6 µl, for example) of liquid.

As shown in Fig. 5, a second recess part 51b is circularly formed on a upper surface of the cartridge body 51 to overlap with an end of the extraction medium holding part 53 (first recess part 51a) on the arrow Y1 direction side in plan view. The liquid storing part 52 is configured by this second recess part 51b. The second recess part 51b is formed to extend downward (in the arrow Z1 direction). As shown in Fig. 4, a second bottom surface 511b of the second recess part 51b is arranged above (on the arrow Z2 direction side) the first bottom surface 512a of the first recess part 51a. In other words, the liquid storing part 52 and the extraction medium holding part 53 are arranged not to overlap with each other in a height direction (the arrow Z1 and arrow Z2 direction). A first passage 56 penetrating to the first bottom surface 512a of the first recess part 51a is formed on a part, where the liquid storing part 52 and the extraction medium holding part 53 overlap with each other in plan view, of the second bottom surface 511b. Thus, the liquid storing part 52 and the extraction medium holding part 53 are spatially connected to each other through the first passage 56. The liquid storing part 52 has a volume capable of storing substantially the same quantity (about 5 µl to about 6 µl, for example) of liquid as the prescribed quantity capable of being held by the extraction medium holding part 53. The liquid storing part 52 stores substantially the same quantity (about 5 µl to about 6 µl, for example) of pure water as the prescribed quantity capable of being held by the extraction medium holding part 53 in a state where the extraction cartridge 50 is unused.

As shown in Figs. 4 and 5, a second passage 57 extending by a prescribed distance from the second recess part 51b in the arrow Y1 direction is formed on the upper surface of the cartridge body 51. The second passage 57 is so formed in a groove shape in a horizontal direction that the upper surface side of the cartridge body 51 is open. A third passage 58 is provided to pass through the cartridge body 51 in a vertical direction (the arrow Z1 direction and the arrow Z2 direction) from an end of the second passage 57 on the arrow Y1 direction side.

As shown in Fig. 4, a through-hole 51c extending in the vertical direction (the arrow Z1 direction and the arrow Z2 direction) is formed at a position opposed to the ejection part 14d of the cartridge body 51 in a state where the extraction cartridge 50 is attached to the extraction cartridge attachment part 14 of the device body 10. The air inflow part 55 is configured by this through-hole 51c. As shown in Figs. 5 and 6, the air inflow part 55 has a circular shape in plan view. The air inflow part 55 is connected to the third passage 58 through a fourth passage 59. The fourth passage 59 is configured similarly to the second passage 57 provided on the upper surface side of the cartridge body 51 and is so formed in a groove shape that the lower surface side of the cartridge body 51 is open. Thus, the air inflow part 55 and the liquid storing part 52 are spatially connected to each other through the second passage 57, the third passage 58 and the fourth passage 59.

The diameter of the circular air inflow part 55 is formed to be smaller than the diameter of the ejection part 14d having a hemisphere shape in plan view. Thus, an opening of the air inflow part 55 on the upper surface side of the cartridge body 51 can be sealed by an outer surface of the ejection part 14d in the state where the extraction cartridge 50 is attached to the extraction cartridge attachment part 14 of the device body 10. At this time, the ejection part 14d is urged by a spring member (not shown) to protrude to the outside of the extraction cartridge attachment part 14, and hence the outer surface of the ejection part 14d and an edge of the opening of the air inflow part 55 come into contact with each other in a state where prescribed urging force is applied thereto. Thus, air ejected from the ejection part 14d can be suppressed from leakage from the opening of the air inflow part 55 on the upper surface side of the cartridge body 51.

As shown in Fig. 5, a third recess part 51d is formed in a rectangular shape on the upper surface of the cartridge body 51 to overlap with an end of the extraction medium holding part 53 (first recess part 51a) on an arrow Y2 direction side in plane view. As shown in Fig. 4, a third bottom surface 511d of the third recess part 51d is arranged above (on the arrow Z2 direction side) the first bottom surface 512a of the first recess part 51a. In other words, the third recess part 51d and the extraction medium holding part 53 are arranged not to overlap with each other in the height direction (the arrow Z1 and arrow Z2 direction). A fifth passage 60 penetrating to the first bottom surface 512a of the first recess part 51a is formed on a part where the third recess part 51d and the extraction medium holding part 53 overlap with each other in plan view, of the third bottom surface 511d. As shown in Fig. 5, a groove part 61 extending from the third recess part 51d to an end of the cartridge body 51 on the arrow Y2 direction side is formed on a central part of the cartridge body 51 on the arrow X1 and arrow X2 direction side. The groove part 61 is so formed that a depth thereof is substantially the same as a depth of the third recess part 51d.

According to the embodiment, as shown in Fig. 5, two light blocking tapes 71 and 72 having prescribed heights are arranged on the third bottom surface 511d of the third recess part 51d to rectangularly surround a substantially central part of the third bottom surface 511d in plan view. More specifically, the first light blocking tape 71 has a rectangular tape, and the second light blocking tape 72 has an L shape. The first light blocking tape 71 is arranged at a position on the arrow X1 direction side with respect to the second light blocking tape 72 and separated by a prescribed distance from an inner side surface of the third recess part 51d. The L-shaped second light blocking tape 72 is arranged at a position on the arrow X2 direction side with respect to the first light blocking tape 71 and separated by a prescribed distance from the inner side surface of the third recess part 51d. A part 72a of the L-shaped light blocking tape 72 extending in the arrow X1 direction is so formed that a forward end thereof is arranged at a position separated by a prescribed distance from the first light blocking tape 71. The light blocking tapes 71 and 72 have a function of blocking light when the detecting part 40 detects glucose.

A sensor member 80 described later is arranged on upper surfaces of the light blocking tapes 71 and 72. The sensor member 80 has a rectangular shape having substantially the same size as the third recess part 51d in plan view and is fitted into the third recess part 51d to block an opening of the third recess part 51d. The light blocking tapes 71 and 72 function as spacers, whereby the sensor member 80 is arranged at a position separated by a prescribed distance (distance substantially equal to heights of the light blocking tapes) from the third bottom surface 511d of the third recess part 51d. The reaction part 54 is configured by being surrounded by the light blocking tapes 71 and 72, the third bottom surface 511d of the third recess part 51d and a lower surface 80a of the sensor member 80 in a substantially central part of the third recess part 51d in plan view. A part of the third recess part 51d other than the reaction part 54 surrounded by the inner side surface of the third recess part 51d, the light blocking tapes 71 and 72, the bottom surface 511d of the third recess part 51d and the lower surface 80a of the sensor member 80 functions as a passage for evacuating air from the reaction part 54 and allowing surplus liquid to escape to the groove part 61. The reaction part 54 is configured to be capable of holding the quantity (about 2.5 µl to about 3 µl, for example) of liquid less than the prescribed quantity capable of being held by the extraction medium holding part 53. A mesh sheet 62 having substantially the same height as the light blocking tapes 71 and 72 is arranged throughout the reaction part 54. Thus, liquid transferred to the reaction part 54 is absorbed by the mesh sheet 62, and hence at least a constant quantity of liquid can be reliably held in the reaction part 54 and the liquid transferred to the reaction part 54 can be easily brought into contact with the lower surface 80a of the sensor member 80.

As shown in Fig. 6, a first electrode 63 and a second electrode 64 are mounted on the lower surface of the cartridge body 51 in the arrow X1 direction and the arrow X2 direction of the opening 511a, respectively. The first electrode 63 and the second electrode 64 are electrically connected to the power supply 16 of the device body 10 through the terminal 14c of the extraction cartridge attachment part 14 and a terminal 51e of the extraction cartridge 50 coming into contact with the terminal 14c in a state where the extraction cartridge 50 is attached to the extraction cartridge attachment part 14 of the device body 10. The first electrode 63 is arranged to extend from an end of the opening 511a on the arrow Y1 direction side to the vicinity of an end of the opening 511a on the arrow Y2 direction side. The second electrode 64 is arranged not to be in contact with the first electrode 63 and extend in the arrow Y1 direction from the end of the opening 511a on the arrow Y2 direction side by a small amount of distance. In other words, the first electrode 63 and the second electrode 64 are arranged to be separated from each other in the vicinity of the end of the opening 511a on the arrow Y2 direction side. The first electrode 63 and the second electrode 64 are arranged at positions actually not appearing in Fig. 4 but are shown in Fig. 4 for convenience of description.

A two-sided tape 65 is attached to a region other than the opening 511a of the extraction medium holding part 53 on the lower surface of the cartridge body 51. Thus, the through-hole 51c and the fourth passage 59 opened on the lower surface side of the cartridge body 51 can be blocked, and the cartridge body 51 can make contact with the subject's arm 110. As shown in Figs. 4 and 5, a tape 66 is attached to the upper surface of the cartridge body 51 to block the second recess part 51b and the second passage 57 opened on the upper surface side of the cartridge body 51.

A part of the lower surface 80a of the sensor member 80 exposed in the reaction part 54 is coated with a mixed gel containing a chromogenic dye pigmenting due to reaction of glucose and prescribed enzymes, and subjected to treatment for drying the mixed gel. More specifically, the lower surface 80a of the sensor member 80 is coated with a mixed gel prepared by mixing glucose oxidase (GOD) which is oxidase serving as a catalyst for glucose, peroxidase (POD) which is oxidoreductase serving as a catalyst for hydrogen peroxide (H₂O₂) resulting from reaction of the glucose through catalytic action of GOD and a chromogenic dye pigmenting by reacting with O^{*} (active oxygen) resulting from reaction of H₂O₂ through catalytic action of POD into a gel, and subjected to treatment for drying the mixed gel.

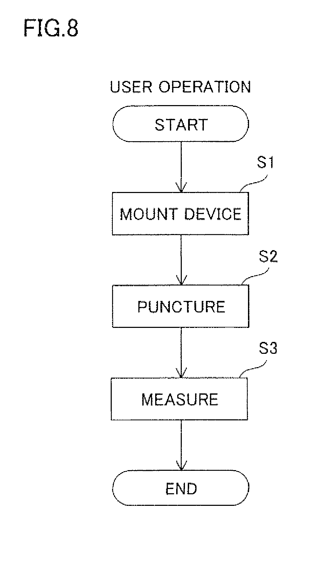

A use procedure of the blood glucose level estimation device 100 according to the embodiment will be now described with reference to Figs. 8 to 11.

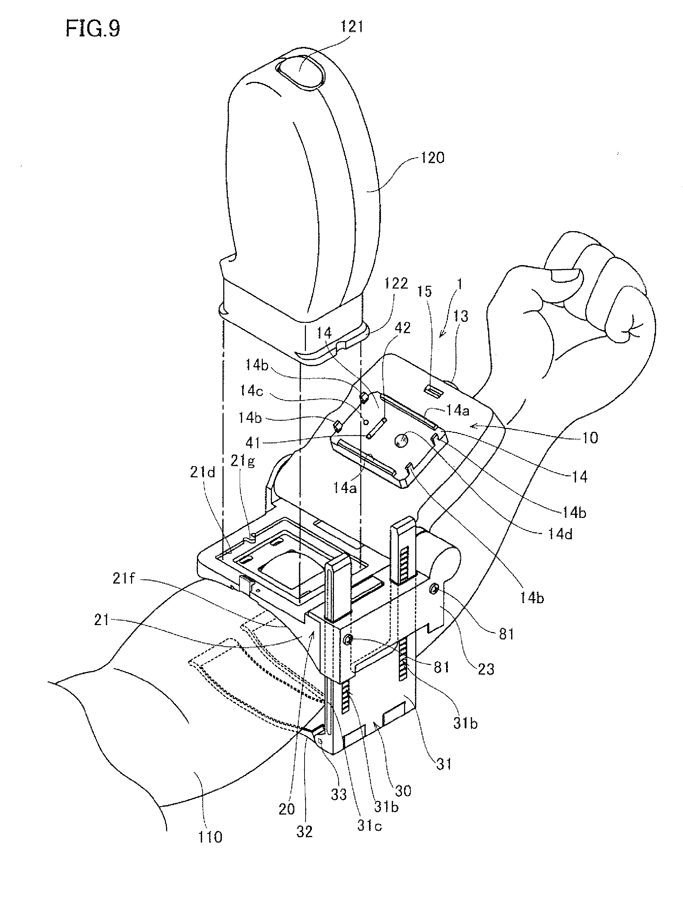

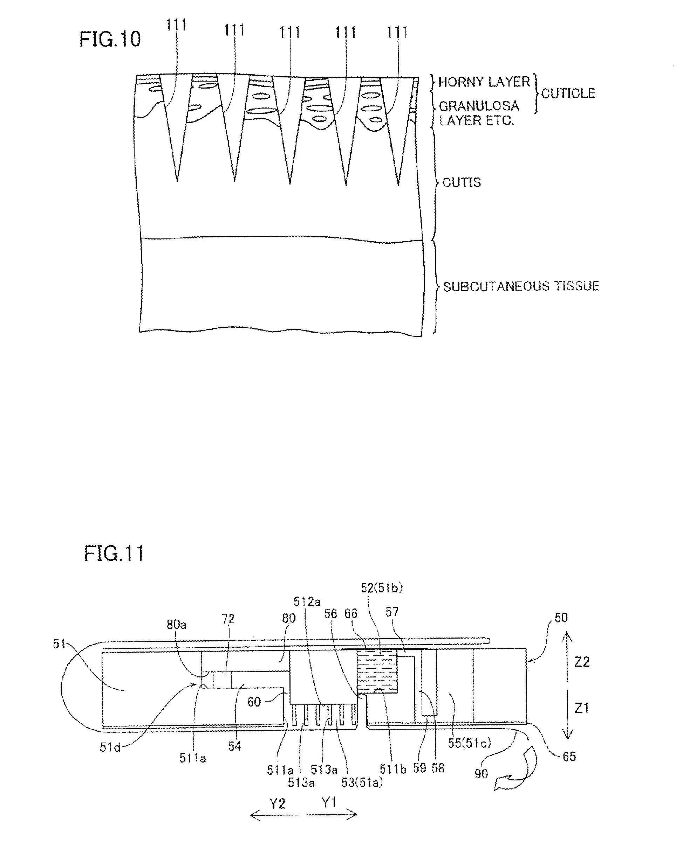

According to the embodiment, as shown in Fig. 9, the blood glucose level estimation device 100 is so mounted on the subject's arm 110 that the subject's writs 110 is tightened between the holding member 20 and the tightening parts 32 of the mounting member 30 at a step S1. Thus, the measuring area 110a is defined by the opening 21a of the holding member 20. At a step S2, micropores (extraction holes) 111 (see Fig. 10) are formed in the measuring area 110a of the subject's arm 110 defined by the opening 21a by the micropore forming device 120. More specifically, after a guide part 122 of the micropore forming device 120 is fitted into the step part 21d of the holding member body 21, minute needles (not shown) protrude from the inside of the micropore forming device 120 by the subject pressing a button part 121. Thus, the micropores (extraction holes) 111 (see Fig. 10) are formed in the measuring area 110a of the subject's arm 110 defined by the opening 21a. At this time, the micropores (extraction holes) 111 passing through the cuticle and reaching the cutis without reaching the subcutaneous tissue can be formed in the subject's arm 110 as shown in Fig. 10, whereby body fluid can be extracted from the subject's arm 110 through the plurality of micropores (extraction holes) 111. Thus, pain felt by the subject can be eased when extracting glucose from the subject's arm 110 with the blood glucose level estimation device 100. After this, the micropore forming device 120 is detached from the holding member body 21.

Next, the extraction cartridge 50 is attached to the extraction cartridge attachment part 14 of the device body 10 at a step S3. More specifically, a film 90 is attached to the unused extraction cartridge 50 to cover a substantially whole area of the upper and lower surfaces of the cartridge body 51, and all the openings exposed on the upper and lower surfaces of the cartridge body 51 are blocked by the film 90, as shown in Fig. 11. Thus, impurities are prevented from adhering to each part provided on the cartridge body 51. The film 90 is welded to an inner side surface of the first passage 56, whereby pure water stored in the liquid storing part 52 is reliably prevented from leaking to the outside and impurities are prevented from being mixed into the pure water. In this state, the subject pulls an end of the film 90 arranged on the arrow Y1 direction side on the lower surface side of the cartridge body 51 in the arrow Y2 direction thereby peeling off the film 90 from the cartridge body 51. At this time, the first passage 56 is opened, but the pure water in the liquid storing part 52 does not leak because of the minute diameter of the first passage 56. Each part except the liquid storing part 52 in the cartridge body 51 is filled with air (gas). The subject mounts the extraction cartridge 50 on the extraction cartridge attachment part 14 of the device body 10. Thereafter, the device body 10 is rotated in a direction B to be rendered in a closed state, and the operation buttons 12 are operated to start measurement.

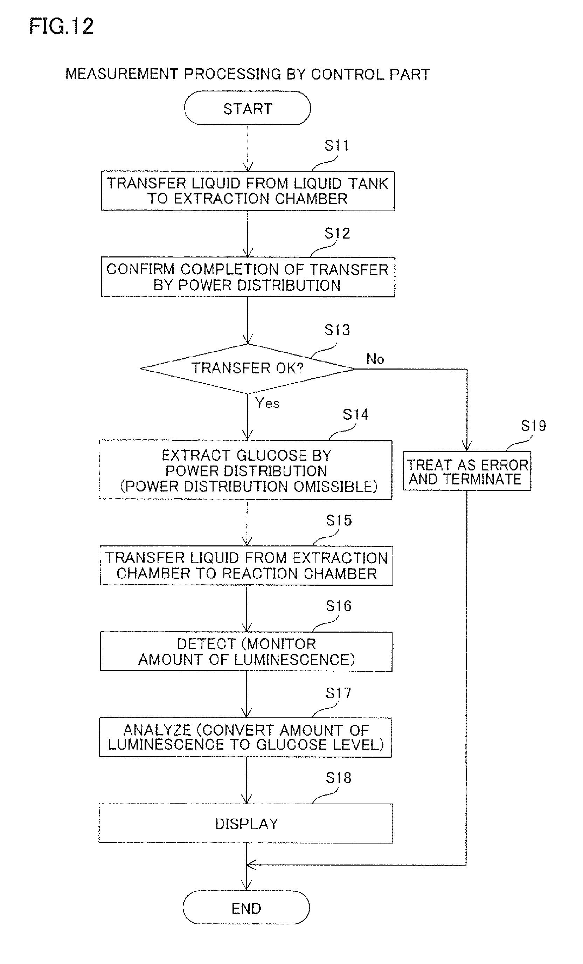

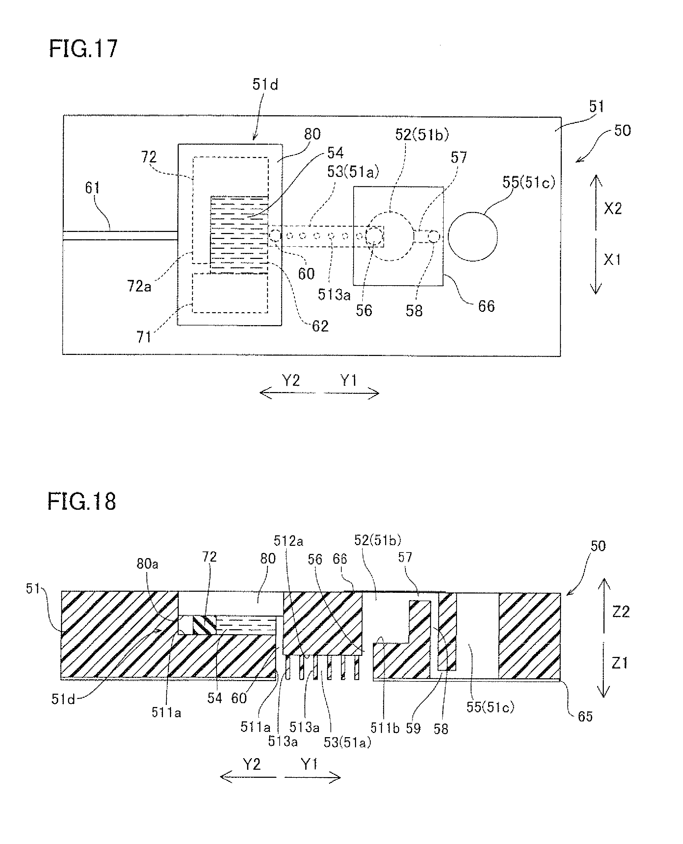

A measurement processing by the control part 17 of the blood glucose level estimation device 100 according to the embodiment will be now described with reference to Figs. 12 to 18.

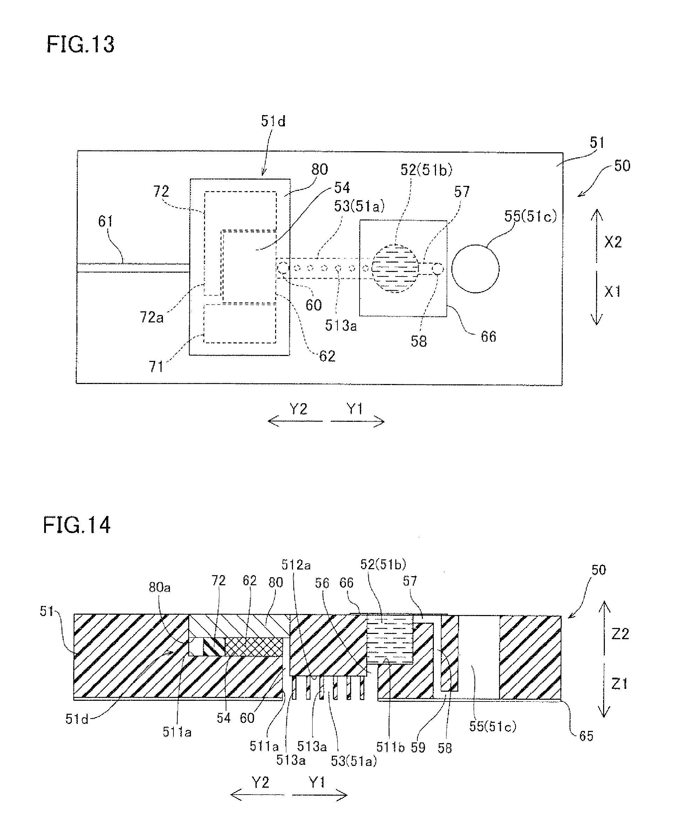

As shown in Figs. 13 and 14, immediately after measurement starts, a prescribed quantity (about 5 µlto about 6 µl, for example) of pure water is stored in the liquid storing part 52. At a step S11, air supplied from the pump 18 is ejected from the ejection part 14d to the air inflow part 55. Thus, air existing in the air inflow part 55, the fourth passage 59, the third passage 58 and the second passage 57 is transferred to the liquid storing part 52, and hence the pure water stored in the liquid storing part 52 is pushed out by the air flowed in from the second passage 57 to be transferred to the extraction medium holding part 53 through the first passage 56. At a step S12, the control part 17 performs an operation for confirming that transfer of the pure water from the liquid storing part 52 to the extraction medium holding part 53 has been completed. More specifically, a high- frequency voltage is applied to the first electrode 63 and the second electrode 64 by the power supply 16, and the control part 17 detects change in impedance between the first electrode 63 and the second electrode 64. In other words, the impedance between the first electrode 63 and the second electrode 64 is reduced when the pure water flowed in the extraction medium holding part 53 from the first passage 56 arranged on the arrow Y1 direction side of the extraction medium holding part 53 reaches a part where the first electrode 63 and the second electrode 64 are separated from each other, in the vicinity of the end of the extraction medium holding part 53 on the arrow Y2 direction side, and hence the control part 17 detects the reduction. Thus, it is possible to confirm that the pure water has been transferred to a substantially whole area of the extraction medium holding part 53.

At a step S13, the control part 17 determines whether or not the transfer of the pure water from the liquid storing part 52 to the extraction medium holding part 53 has been completed on the basis of the detection result of the change in the impedance between the first electrode 63 and the second electrode 64. The control part 17 may be configured to determine that the transfer of the pure water from the liquid storing part 52 to the extraction medium holding part 53 has been completed when the impedance between the first electrode 63 and the second electrode 64 falls below a prescribed threshold or when the amount of reduction in the impedance within a prescribed time exceeds a threshold in this determination. When the transfer has not been completed even after a lapse of the prescribed time, the same is treated as an error at a step S19, and the measurement process operation is terminated.

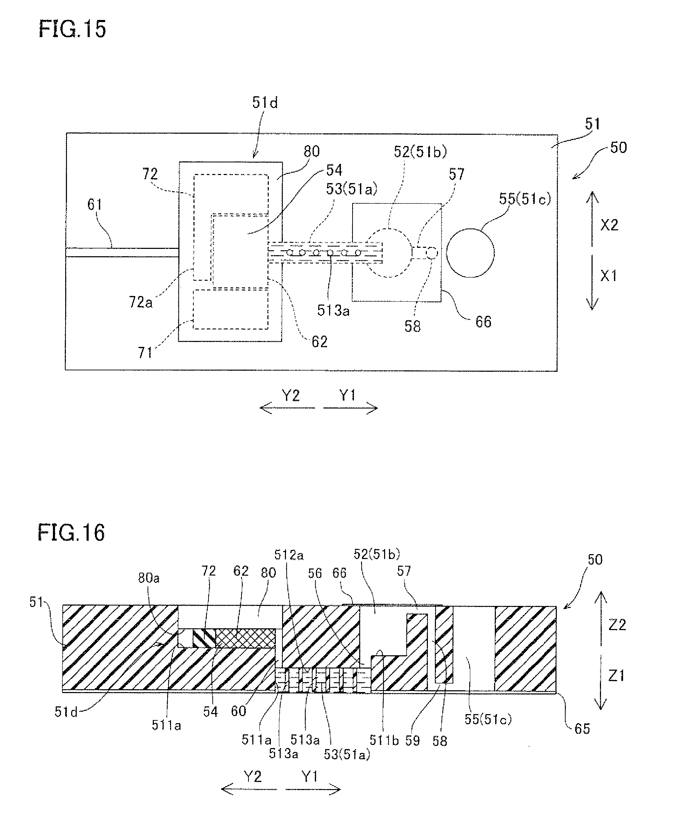

When the control part 17 determines that the transfer of the pure water has been completed, on the other hand, the supply of air from the pump 18 is stopped and the transfer of the pure water is stopped. At this time, the prescribed quantity (about 5 µl to about 6 µl, for example) of the pure water exists only in the extraction medium holding part 53, as shown in Figs. 15 and 16 (in other words, a region where the pure water exists is the extraction medium holding part 53 in Figs. 15 and 16). At a step S14, glucose is extracted from the subject's arm 110 (micropores 111) for a prescribed time in a state where the pure water is held in the extraction medium holding part 53, the opening 511a of which is blocked by the surface of the subject's arm 110. More specifically, a constant current is so supplied by the power supply 16 that the first electrode 63 and the second electrode 64 serve as negative electrodes and the sheet made of a conductive material attached to the inner surface of the mounting member 30 serves as a positive electrode on the basis of control by the control part 17. Thus, glucose in the subject's body easily moves toward the first electrode 63 and the second electrode 64 applied with a negative voltage, and hence glucose can be smoothly extracted from the subject's arm 110 (micropores 111) to the pure water in the extraction medium holding part 53. After a lapse of the prescribed time, the supply of the constant current from the power supply 16 to the first and second electrodes 63 and 64 and the sheet, made of a conductive material, of the mounting member 30 is stopped, and the extraction of glucose is completed. The power supply 16 may supply a direct current, an alternating current or a mixed current of a direct current and an alternating current. The power supply 16 may be a constant-voltage power supply applying a constant voltage.

Thereafter, at a step S15, the supply of air from the pump 18 restarts. Thus, air existing in the air inflow part 55, the fourth passage 59, the third passage 58, the second passage 57 and the liquid storing part 52 is transferred to the extraction medium holding part 53, and hence part (about 2.5 µl to about 3 µl, for example) of the pure water (this liquid is hereinafter referred to as glucose holding liquid) in the extraction medium holding part 53, holding the extracted glucose is pushed out by the air flowed in from the first passage 56 to be transferred to the reaction part 54 through the fifth passage 60. The glucose holding liquid transferred to the reaction part 54 is absorbed by the mesh sheet 62 set in the reaction part 54 thereby being held in the reaction part 54 and coming into contact with the mixed gel applied to the lower surface 80a of the sensor member 80. Air having existed in the reaction part 54 before the transfer of the glucose holding liquid is released to the outside of the extraction cartridge 50 through the groove part 61 following the transfer of the glucose holding liquid. The surplus glucose holding liquid other than the glucose holding liquid transferred to the reaction part 54 is allowed to escape to a space (passage) other than the reaction part 54 in the third recess part 51d.

At a step S16, detection of glucose starts. More specifically, the control part 17 continuously monitors absorbance when light of the light source part 41 is absorbed by a chromogenic dye of the sensor member 80 for a prescribed time by employing the light source part 41 and the photoreceptor 42 configuring the detecting part 40 (see Fig. 4). Then, at a step S17, the control part 17 calculates the blood glucose level on the basis of absorbance when the absorbance becomes substantially saturated. At a step S18, the calculated blood glucose level is displayed on the display 11, and the measurement processing operations by the control part 17 are terminated.

According to this embodiment, as hereinabove described, the blood glucose level estimation device 100 is provided with the extraction medium holding part 53 configured to be capable of holding the prescribed quantity of extraction medium (pure water) for holding glucose extracted from the subject's arm 110, the reaction 54 allowing the glucose to cause the prescribed reaction and the pump 18 for transferring at least a part of the extraction medium holding the glucose extracted from the subject's arm 110 from the extraction medium holding part 53 to the reaction part 54, whereby the reaction part 54 can be held in a dry state until the extraction medium holding the glucose is transferred from the extraction medium holding part 53 to the reaction part 54, and hence the catalyst can be suppressed from elution into the extraction medium also when the catalyst for glucose is provided in the reaction part 54. Thus, analytical accuracy of the glucose by the control part 17 can be suppressed from reduction. In this blood glucose level estimation device 100, the liquid storing part 52 storing substantially the same quantity of extraction medium as the prescribed quantity capable of being held by the extraction medium holding part 53 is provided and the pump 18 is controlled to transfer the extraction medium stored in the liquid storing part 52 to the extraction medium holding part 53 capable of holding the prescribed quantity of extraction medium, whereby glucose can be extracted in a state where the extraction medium exists substantially only in the extraction medium holding part 53. Therefore, the glucose held by the extraction medium can be prevented from mixing with the extraction medium outside the extraction medium holding part 53. Thus, the concentration of the glucose can be suppressed from becoming non-uniform, and hence the glucose extracted from the subject's arm 110 can be more accurately analyzed.

According to this embodiment, as hereinabove described, the prescribed quantity of extraction medium capable of being held by the extraction medium holding part 53 is rendered larger than the quantity of extraction medium capable of being held by the reaction part 54, whereby the reaction part 54 can be filled with the extraction medium holding an extract, and hence the extract can be accurately detected by the detecting part 40. Consequently, the extract can be more accurately analyzed by the control part 17.

According to this embodiment, as hereinabove described, the first passage 56 and the second passage 57 each filled with gas and connected to the liquid storing part 52 are provided, whereby the extraction medium (pure water) stored in the liquid storing part 52 is not pushed out (transferred) to the extraction medium holding part 53 through liquid such as the extraction medium (pure water) but can be pushed out (transferred) to the extraction medium holding part 53 through the gas in the second passage 57, and hence the extraction medium transferred to the extraction medium holding part 53 can be prevented from coming into contact with the liquid outside the extraction medium holding part 53.

According to this embodiment, as hereinabove described, the air inflow part 55 for allowing air to flow in the second passage 57 and the ejection part 14d configured to be capable of blocking the air inflow part 55, ejecting the air supplied from the pump 18 to the air inflow part 55 are provided, whereby the air can be suppressed from leakage from between the air inflow part 55 and the ejection part 14d, and hence the extraction medium stored in the liquid storing part 52 can be reliably flowed in the extraction medium holding part 53.

According to this embodiment, as hereinabove described, the extraction cartridge 50 including the extraction medium holding part 53, the reaction part 54 and the liquid storing part 52 and the device body 10 including the detecting part 40, the pump 18 and the control part 17, configured to allow the extraction cartridge 50 to be attached and detached are provided, whereby only the extraction cartridge 50 including the extraction medium holding part 53, the reaction part 54 and the liquid storing part 52 can be easily replaced.

According to this embodiment, as hereinabove described, the first electrode 63 and the second electrode 64 promoting extraction of the extract are provided, whereby the extraction of the extract can be performed in a shorter time, and hence an analytical result of the extract can be obtained in a shorter time.

According to this embodiment, as hereinabove described, the intrusion prevention parts preventing intrusion of the skin of the subject's arm 110 into the extraction medium holding part 53 are provided in the extraction medium holding part 53, whereby the volume of the extraction medium holding part 53 can be prevented from reducing by the intrusion of the skin of the subject's arm 110 into the extraction medium holding part 53, and hence the extraction medium transferred from the liquid storing part 52 to the extraction medium holding part 53 can be suppressed from overflowing outside the extraction medium holding part 53. Thus, the volume of the extraction medium holding part 53 can be prevented from change, and hence the quantity of extraction medium in the extraction medium holding part 53 can be kept constant. Consequently, change in the concentration of the extract resulting from change in the quantity of extraction medium can be suppressed, and hence the glucose extracted from the subject's arm 110 can be more accurately analyzed.

According to this embodiment, as hereinabove described, the control part 17 analyzing the glucose (extract) is configured to analyze the detection result by the detecting part 40 and acquire the blood glucose level, whereby the blood glucose level based on the detection result of the extracted glucose can be estimated.

According to this embodiment, as hereinabove described, this blood glucose level estimation device 100 is provided with the first passage 56 and the second passage 57 filled with gas and connected to the extraction medium holding part 53, whereby the extraction medium in the extraction medium holding part 53 does not come into contact with the liquid outside the extraction medium holding part 53, and hence the glucose held by the extraction medium can be prevented from mixing with the liquid outside the extraction medium holding part 53. Thus, the concentration of the glucose can be suppressed from becoming non-uniform, and hence the glucose extracted from the subject's arm 110 can be more accurately analyzed.

The embodiment disclosed this time must be considered as illustrative in all points and not restrictive. The range of the present invention is shown not by the above description of the embodiment but by the scope of claims for patent, and all modifications within the meaning and range equivalent to the scope of claims for patent are further included.

For example, while the blood glucose level estimation device has been shown as an example of the biological component analyzer in the aforementioned embodiment, the present invention is not restricted to this, but the present invention can also be applied to another biological component analyzer.

While the example of forming the micropores (extraction holes) in the subject's arm by the micropore forming device on the basis of the subject's operation and promoting the extraction of the glucose by applying a voltage to the electrodes has been shown in the aforementioned embodiment, the present invention is not restricted to this, but either the aforementioned step of forming the micropores (extraction holes) in the subject's arm or the aforementioned step of promoting the extraction of the glucose by applying a voltage to the electrodes may be omitted.

While the example of the structure transferring the extraction medium from the liquid storing part to the extraction medium holding part has been shown in the aforementioned embodiment, the present invention is not restricted to this, but the aforementioned prescribed quantity (about 5 µl to about 6 µl, for example) of extraction medium may be previously stored in the extraction medium holding part. In this case, no liquid storing part may be provided, and the shape of the cartridge is not limited so far as an entrance passage (fifth passage) and an exit passage (first passage) of the extraction medium holding part are filled with gas.

While the example of the structure starting measurement by the subject's operation of the operation buttons of the device body has been shown in the aforementioned embodiment, the present invention is not restricted to this, but the blood glucose level estimation device may be configured to automatically start measurement when the device body is closed, without the subject's operation of the operation buttons.

While the example of providing the columnar intrusion prevention parts in the extraction medium holding part has been shown in the aforementioned embodiment, the present invention is not restricted to this, but mesh-shaped intrusion prevention parts may be provided in place of the columnar intrusion prevention parts, for example, or no intrusion prevention parts may be provided.

While the example of the structure pushing out the extraction medium stored in the liquid storing part through air (gas) has been shown in the aforementioned embodiment, the present invention is not restricted to this, but the extraction medium may be directly pushed out by a pressing member without employing air, or the extraction medium may be pushed out through liquid not mixed with the extraction medium, for example.

## Claims

1. A method of analyzing a biological component comprising steps of:
arranging an extraction medium holding part configured to be capable of holding a prescribed quantity of extraction medium for holding an extract extracted from a subject on the skin of the subject;
transferring said extraction medium stored in a liquid storing part storing substantially the same quantity of said extraction medium as said prescribed quantity to said extraction medium holding part;
extracting said extract to said extraction medium transferred to said extraction medium holding part;
transferring at least part of said extraction medium holding extracted said extract to a reaction part allowing said extract to cause a prescribed reaction;
detecting said extract from said prescribed reaction caused in said reaction part; and
analyzing a result of detecting said extract.

2. The method of analyzing a biological component according to claim 1, wherein
said step of transferring said extraction medium to said extraction medium holding part includes a step of transferring said extraction medium stored in said liquid storing part to said extraction medium holding part with a liquid transfer part.

3. The method of analyzing a biological component according to claim 2, wherein
said step of transferring said extraction medium with said liquid transfer part includes a step of transferring said extraction medium stored in said liquid storing part to said extraction medium holding part by pressure of air by allowing air to flow in an entrance passage provided between said liquid transfer part and said liquid storing part and filled with air by said liquid transfer part.

4. The method of analyzing a biological component according to claim 3, wherein
said step of transferring said extraction medium to said extraction medium holding part with said liquid transfer part includes a step of transferring substantially all of said extraction medium stored in said liquid storing part to said extraction medium holding part through an exit passage provided between said liquid storing part and said extraction medium holding part.

5. The method of analyzing a biological component according to claim 1, wherein
said step of transferring at least said part of said extraction medium to said reaction part includes a step of transferring at least said part of said extraction medium holding extracted said extract to said reaction part with said liquid transfer part.

6. The method of analyzing a biological component according to claim 1, further comprising a step of allowing a mesh sheet arranged in said reaction part to absorb said extraction medium transferred to said reaction part after said step of transferring at least said part of said extraction medium to said reaction part.

7. The method of analyzing a biological component according to any one of claims 1 to 6, wherein
said step of detecting said extract includes a step of detecting glucose, and
said step of analyzing said result of detecting said extract includes a step of acquiring a blood glucose level.

8. A biological component analyzer, comprising:
an extraction medium holding part capable of holding a prescribed quantity of extraction medium for holding an extract extracted from a subject;
a reaction part allowing said extract to cause a prescribed reaction;
a detecting part detecting said extract from said prescribed reaction caused in said reaction part;
a liquid storing part storing substantially the same quantity of said extraction medium as said prescribed quantity;
a liquid transfer part transferring said extraction medium stored in said liquid storing part to said extraction medium holding part and transferring at least part of said extraction medium transferred to said extraction medium holding part, holding said extract extracted from said subject from said extraction medium holding part to said reaction part; and
an analysis part analyzing a result of detecting said extract,
being configured to allow for extraction of said extract to said extraction medium holding part by arranging said extraction medium holding part to be in contact with the skin of said subject.

9. The biological component analyzer according to claim 8, wherein
said prescribed quantity of said extraction medium capable of being held by said extraction medium holding part is larger than the quantity of said extraction medium capable of being held by said reaction part.

10. The biological component analyzer according to claim 8 or 9, further comprising an entrance passage and an exit passage each filled with gas and connected to said liquid storing part.

11. The biological component analyzer according to claim 10, wherein
said gas is air,
the biological component analyzer further comprising:
an air inflow part for allowing air to flow in said entrance passage; and
an ejection part configured to be capable of blocking said air inflow part, ejecting air supplied from said liquid transfer part to said air inflow part.

12. The biological component analyzer according to any one of claims 8 to 11, further comprising:
a cartridge including said extraction medium holding part, said reaction part and said liquid storing part; and
an analyzer body including said detecting part, said liquid transfer part and said analysis part, configured to allow said cartridge to be attached and detached.

13. The biological component analyzer according to any one of claims 8 to 12, further comprising an extraction promoting part promoting extraction of said extract.

14. The biological component analyzer according to any one of claims 8 to 13, further comprising an intrusion prevention part preventing the skin of said subject from intrusion into said extraction medium holding part.

15. The biological component analyzer according to any one of claims 8 to 14, further comprising a mesh sheet arranged in said reaction part, capable of absorbing at least part of said extraction medium transferred to said reaction part.

16. The biological component analyzer according to any one of claims 8 to 15, wherein
said extract is glucose, and
said analysis part is configured to estimate a blood glucose level by analyzing a result of detecting said glucose.

17. A biological component analyzer, comprising:
an extraction medium holding part holding a prescribed quantity of extraction medium for holding an extract extracted from a subject;
an entrance passage and an exit passage filled with gas, connected to said extraction medium holding part;
a reaction part allowing said extract to cause a prescribed reaction;
a detecting part detecting said extract from said prescribed reaction caused in said reaction part;
a liquid transfer part transferring at least part of said extraction medium holding said extract extracted from said subject from said extraction medium holding part to said reaction part through said exit passage; and
an analysis part analyzing a result of detecting said extract,
being configured to allow for extraction of said extract to said extraction medium holding part by arranging said extraction medium holding part to be in contact with the skin of said subject.

18. An extraction cartridge, comprising:
an extraction medium holding part configured to be capable of holding a prescribed quantity of extraction medium for holding an extract extracted from a subject;
a reaction part allowing said extract to cause a prescribed reaction; and
a liquid storing part storing substantially the same quantity of said extraction medium as said prescribed quantity,
being detachable on an analyzer body detecting said extract from said prescribed reaction caused in said reaction part to analyze the same.

19. The extraction cartridge according to claim 18, further comprising a mesh sheet arranged in said reaction part, capable of absorbing at least part of said extraction medium transferred to said reaction part.

20. An extraction cartridge, comprising:
an extraction medium holding part holding a prescribed quantity of extraction medium for holding an extract extracted from a subject;
an entrance passage and an exit passage filled with gas, connected to said extraction medium holding part; and
a reaction part allowing said extract to cause a prescribed reaction,
being detachable on an analyzer body detecting said extract from said prescribed reaction caused in said reaction part to analyze the same.
